# EUROPEAN PATENT APPLICATION

(11) **EP 2 659 899 A2**
(43) Date of publication of application: **06.11.2013**
(21) Application number: 13160707.9
(22) Date of filing: 22.03.2013
(51) Int. Cl.: A61K 36/185, A61K 36/234, A61K 36/30, A61K 36/65, A61P 17/00, A61P 17/16

(54) **Lipophilic carrier composition for solubilizing lipophilic bioactive botanical extracts, methods of solubilizing lipophilic bioactive botanical extracts, and methods of using solubilized lipophilic bioactive botanical extracts**

(30) Priority: 23.03.2012 US 201261614838 P; 13.06.2012 US 201213495754
(71) Applicant: Innovacos Corporation, Mt Arlington, New Jersey 07856 (US)
(72) Inventor: Thibodeau, Alain, Québec G3A 2V8 (CA)
(74) Representative: Pistolesi, Roberto

(57) **Abstract**

A lipophilic carrier composition comprising a lipophilic bioactive botanical extract, a vegetable oil, a solubilization system, and, optionally, an antioxidant, where the vegetable oil is present in a stable, relatively unoxidized state, is provided. Also provided is a lipophilic antioxidant composition comprising an antioxidant, a vegetable oil, and a solubilization system, but not comprising a lipophilic bioactive botanical extract, where the vegetable oil is present in a stable, relatively unoxidized state. Disclosed are methods of using the compositions to treat and/or protecting skin against erythema and/or skin barrier function loss as well as methods of using the compositions to treat age spots.

## Description

Skin is a complex living tissue, which is composed of two main layers: the dermis and the epidermis. The epidermis is the outer layer of the skin, and is organized in layers of cells called keratinocytes that progressively differentiate to form the outermost layer of the epidermis, known as the stratum corneum. Fully differentiated keratinocytes, known as corneocytes, are devoid of nuclei and are filled with insoluble keratin fibers. They are arranged like bricks and separated by lipid-rich layers in an array that is often referred to as the brick-and-mortar model of the stratum corneum. In addition to protecting the inner layers of the skin from deleterious chemicals and harmful radiation, the stratum corneum acts as a barrier to the passage of hydrophilic compounds. Metabolically active keratinocytes, which form the remainder of the epidermis, and cells that respond to external stimuli are located below the stratum corneum. The dermis lies below the epidermis and is constituted mainly of fibroblasts, which are metabolically active cells that can respond to signals coming from upper layers of the skin and from the external environment.

Bioactive botanical extracts, having beneficial effects on the skin, e.g., photoprotection, anti-aging, moisturizing, antioxidant, astringent, anti-irritant, and antimicrobial properties, are being increasingly used in the cosmetic industry and are featured in a growing variety of cosmetic formulations and products available in the marketplace. Such bioactive botanical extracts and mixtures of botanical extracts are often obtained by extracting biomasses in solvents that are compatible with cosmetic uses. For safety, environmental and economic reasons, water is generally the extraction solvent of choice and the extractions result in water soluble, or hydrophilic, biological active ingredients. Generally these extracts are formulated in a cosmetically acceptable carrier for appl ication to the skin. Because they have been obtained through water extraction, these extracts are hydrophilic and can be easily added to hydrophilic gels and toiletries or to the water phase of an emulsion. However, because of their hydrophilic nature, the active ingredients in these extracts may have difficulty penetrating the lipophilic stratum corneum barrier of the skin. This decreased penetration generally is thought to lead to decreased efficacy. While means for facilitating the passage of hydrophilic active ingredients through the stratum corneum's lipophilic barrier exist, e.g., incorporation into liposomes or other vehicles having lipophilic characteristics, these means generally involve additional transformation of the active ingredients and may affect their efficacy.

The cosmetics industry is therefore becoming more interested in lipophilic bioactive botanical extracts. Due to their lipophilic nature and enhanced physiological compatibility relative to hydrophilic compounds, lipophilic bioactive extracts are thought to have enhanced ability to penetrate the skin's lipophilic stratum corneum barrier and thus improved biological efficacy resulting from a better ability to reach the metabolically active cells in the dermis and lower layers of the epidermis.

Lipophilic bioactive botanical extracts are known. Unfortunately, because of their physico-chemical properties, in particular their lipophilic nature, many of such lipophilic bioactive botanical extracts have the disadvantage that they do not lend themselves to easy incorporation in an effective amount into cosmetic formulations because, *inter alia*, they are not readily soluble in cosmetic formulation media and their solubilization requires conditions, such as excessive processing, *e.g*., stirring, sonication or mixing, elevated temperatures or aggressive solvents, that often result in their degradation, or conditions that are not compatible with cosmetic uses, or conditions that may be deleterious to other compounds present in the formulation.

Another major challenge in providing high quality cosmetic products based on "natural" ingredients such as bioactive botanical extracts is their poor stability in formulation. Many bioactive botanical extracts do not tolerate processing and storage conditions that are generally acceptable for more stable synthetic ingredients.

A further challenge in providing high quality cosmetic products using "natural" ingredients is the poor stability of certain vegetable oils used in cosmetic formulations. Due to their poor stability, unsaturated vegetable oils do not tolerate processing conditions, storage conditions, or the presence of other reactive ingredients in formulations that are generally acceptable for more stable synthetic ingredients. For example, some seed derived polyunsaturated oils, those containing essential fatty acids in particular, have been shown to improve the structure and function of cell membranes, improve skin barrier function and enhance skin penetration. However, because these oils contain high amounts of unsaturated fatty acids, which are in part responsible for those properties, they are also very vulnerable to oxidation. The labile olefin moiety characteristic of these oils is very easily oxidized upon exposure to oxygen, especially at elevated temperatures. As a result, it is often the case that, by the time products containing these oils reach the consumer, the benefits of such oils have been lost. Although microencapsulation technology has been proposed to protect such oils from oxidation, investment in new apparatus is generally required to utilize such technology and the process is generally time consuming and expensive. Furthermore, depending upon the microencapsulation used, the skin bioavailability of oil components may be hampered.

There remains a need in the cosmetics industry to provide a means of incorporating lipophilic bioactive botanical extracts into a variety of topical dermatological, pharmaceutical and cosmetic preparations that is cost-effective and compatible with standard equipment while preserving the integrity and beneficial properties of these materials, particularly the beneficial properties of the oil components of these materials.

The present invention provides a simple and cost-effective solution to this need by providing a lipophilic bioactive botanical extract presolubilized in an oxidation stable and cosmetically acceptable vegetable oil carrier (i.e., a lipophilic carrier composition). The inventive lipophilic carrier composition is oxidation stable and can readily be incorporated into a variety of cosmetic formulations, improving the processing time and providing a more shelf stable product.

### SUMMARY OF THE INVENTION

In one embodiment, the present invention provides a lipophilic carrier composition comprising a lipophilic bioactive botanical extract, a vegetable oil, a solubilization system, and, optionally, an antioxidant, where the vegetable oil is present in a stable, relatively unoxidized state. In certain embodiments, the vegetable oil is resistant to oxidation such that the Rancimat induction time for the lipophilic carrier composition according to ISO Method no. 6886-2006 is greater than 3 hours, greater than 4 hours, greater than 5 hours, greater than 7 hours, greater than 10 hours, greater than 15 hours, or greater than 20 hours.

In another embodiment, the present invention provides a lipophilic carrier composition comprising a lipophilic bioactive botanical extract, a vegetable oil, a solubilization system, and, optionally, an antioxidant, where the lipophilic carrier composition is produced by a method comprising:
(a) combining the solubilization system and the lipophilic bioactive botanical extract to form a mixture;
(b) maintaining the temperature of the mixture between 40-100°C under a nitrogen or other inert gas atmosphere until the lipophilic bioactive botanical extract is dissolved;
(c) cooling the mixture of step (b);
(d) adding the vegetable oil; and
(e) agitating the mixture formed by the addition of the vegetable oil in step (d) until a homogeneous composition is obtained.

In some embodiments, the mixture of step (c) is cooled to about room temperature.

If the antioxidant is part of the lipophilic carrier composition, it may be added along with the vegetable oil in step (d) or it may be added after the vegetable oil but before step (e). Generally, the antioxidant is dissolved in a cosmetically acceptable alcohol before addition.

In another embodiment, the present invention provides a lipophilic antioxidant composition comprising an antioxidant, a vegetable oil, and a solubilization system, but not comprising a lipophilic bioactive botanical extract, where the vegetable oil is present in a stable, relatively unoxidized state. In certain embodiments, the vegetable oil is resistant to oxidation such that the Rancimat induction time for the lipophilic antioxidant composition according to ISO Method no. 6886-2006 is greater than 3 hours, greater than 4 hours, greater than 5 hours, greater than 7 hours, greater than 10 hours, greater than 15 hours, or greater than 20 hours.

In another embodiment, the present invention provides a lipophilic antioxidant composition comprising an antioxidant, a vegetable oil, and a solubilization system, but not comprising a lipophilic bioactive botanical extract, where the lipophilic antioxidant composition is produced by a method comprising:
(a) combining the solubilization system and the antioxidant to form a mixture;
(b) maintaining the mixture at a temperature that is higher than room temperature under a nitrogen or other inert gas atmosphere such that the antioxidant is dissolved;
(c) reducing the temperature of the mixture of step (b) to room temperature;
(d) adding the vegetable oil to the mixture of step (c); and
(e) agitating the mixture formed by the addition of the vegetable oil in step (d) until a homogeneous composition is obtained.

In some embodiment, the temperature that is higher than room temperature of step (b) is between 40-100°C. In some embodiments, the temperature that is higher than room temperature of step (b) is between 40-50°C, between 50-60°C, between 60-70°C, between 70-80°C, between 80-90°C, or between 90-100°C.

In certain embodiments, the solubilization system comprises a branched, long chain alcohol such as octyldodecanol, a cosmetically acceptable alcohol such as ethanol, and one or more fatty acid esters of a branched, long chain alcohol, for example, octyldodecyl oleate and/or octyldodecyl stearoyl stearate. In certain embodiments, the branched, long chain alcohol in the fatty acid esters is the same branched, long chain alcohol as the unesterified branched, long chain alcohol. In other embodiments, the branched, long chain alcohol in the fatty acid esters is a different branched, long chain alcohol from the unesterified branched, long chain alcohol. In certain embodiments, the fatty acids of the esters are resistant to oxidation. For example, in certain embodiments, the fatty acids do not contain a double bond. In other embodiments, the fatty acids contains only one double bond. Suitable fatty acids include monounsaturated fatty acids such as myristoleic acid (14:1), palmitoleic acid (16:1), sapienic acid (16:1), oleic acid (18:1), elaidic acid (18:1), vaccenic acid (18:1), eicosenoic acid (20:1), and erucid acid (22:1). Other suitable fatty acids include saturated fatty acids such as caprylic acid (8:0), capric acid (10:0), lauric acid (12:0), myristic acid (14:0), palmitic acid (16:0), stearic acid (18:0), arachidic acid (20:0), behenic acid (22:0), lignoceric acid (24:0), and cerotic acid (26:0).

The solubilization system enables the complete dissolution of lipophilic bioactive botanical extracts therein. The solubilization system thus allows for the convenient incorporation of lipophilic active ingredients such as those found in lipophilic bioactive botanical extracts into a vegetable oil, thus forming a lipophilic carrier composition comprising the solubilization system, lipophilic bioactive botanical extract, and vegetable oil, without exposing the vegetable oil to elevated temperatures or extended processing time. As is known in the art, elevated temperatures and extended processing times can lead to the undesired oxidation of vegetable oils. Thus, the lipophilic carrier composition of the present invention contains the vegetable oil in a stable, relatively unoxidized state. The ability to provide lipophilic bioactive botanical extracts in combination with a stable, relatively unoxidized vegetable oil is an advantageous feature of the present invention.

The lipophilic carrier composition can conveniently be used to store and distribute lipophilic active ingredients and to formulate topical cosmetic, pharmaceutical, and dermatologic formulations while maintaining the bioactivity of the lipophilic bioactive botanical extract and preventing oxidation of the vegetable oil and other oxidizable components of the formulation made from the lipophilic carrier composition.

A presolubilized lipophilic bioactive botanical extract for use in the present invention may be made by a method comprising combining a solubilization system with a lipophilic bioactive botanical extract and, if necessary to dissolve the lipophilic bioactive botanical extract, heating the mixture to a temperature between 40-100°C under a nitrogen or other inert gas atmosphere. In some embodiments, the mixture is heated to a temperature between 40-50°C, between 50-60°C, between 60-70°C, between 70-80°C, between 80-90°C, or between 90-100°C. In some embodiments, the inert gas is selected from the group consisting of noble gases such as argon, xenon, neon, and helium. In some embodiments, a mixture of nitrogen and another inert gas is used. In alternative embodiments, rather than using an inert gas to prevent oxidation, a vacuum is used to prevent contact with air, and thus prevent oxidation.

The present invention also provides a method of making a lipophilic carrier composition comprising combining the solubilization system with the lipophilic bioactive botanical extract and, if necessary to dissolve the lipophilic bioactive botanical extract, heating the mixture to a temperature between 40-100°C under a nitrogen or other inert gas atmosphere until the extract is dissolved, then cooling the mixture to room temperature, adding a vegetable oil, and then, optionally, adding an antioxidant extract dissolved in a cosmetically acceptable alcohol. The mixture is then agitated until a homogeneous composition is obtained.

The lipophilic active carrier compositions of the invention can be used to prepare cosmetics having extended shelf life, for the convenient storage and distribution of a lipophilic biological extract, for use as a base or ingredient in a variety of cosmetic compositions, and for preserving the biological activity of additional ingredients included in products produced using the inventive lipophilic active carrier composition (e.g., unsaturated fatty acids, polyphenols, additional liposoluble actives).

The invention also provides a method of protecting skin against erythema and/or skin barrier function loss due to exposure of the skin to radiation or chemical stress using the inventive lipophilic active carrier compositions disclosed herein. Such methods of protecting skin generally involve applying a cosmetic or pharmaceutical formulation produced by incorporating a lipophilic carrier composition of the present invention into a base cream before the insult that results in erythema and/or skin barrier function loss. Thus, included in the present invention are methods of protecting skin against erythema and/or skin barrier function loss by applying a cosmetic or pharmaceutical formulation produced by incorporating a lipophilic carrier composition of the present invention into a base cream to skin that is at risk of suffering radiation or chemical stress. Such "at risk" skin might include, e.g., the skin of a person who contemplates exposing his or her skin to a significant amount of strong sunlight, e.g., by spending a day at the beach. Other "at risk" skin might include the skin of a person who is exposed to chemical stress (e.g., by occupational exposure to chemical aggressors or by frequent use of soaps or cleansers).

A method of treating skin that has developed erythema and/or skin barrier function loss due to exposure of the skin to radiation or chemical stress using the lipophilic active carrier compositions disclosed herein is also provided. Such methods of treating skin generally involve applying a cosmetic or pharmaceutical formulation produced by incorporating a lipophilic carrier composition of the present invention into a base cream after the insult that results in erythema and/or skin barrier function loss.

Of course, cosmetic or pharmaceutical formulations produced by incorporating a lipophilic carrier composition of the present invention into a base cream may be utilized by applying such formulations to the skin both before and after an insult.

The invention also provides methods of treating age spots using the inventive lipophilic active carrier compositions disclosed herein. Such methods of treating age spots generally involve applying a cosmetic or pharmaceutical formulation produced by incorporating a lipophilic carrier composition of the present invention into a base cream to an area of skin containing age spots. Thus, included in the present invention are methods of treating age spots by applying a cosmetic or pharmaceutical formulation produced by incorporating a lipophilic carrier composition of the present invention into a base cream to skin containing age spots. In some embodiments, the cosmetic or pharmaceutical formulation is applied to an area of skin including the age spots and surrounding areas of skin. In some embodiments, the cosmetic or pharmaceutical formulation is applied directly to the age spots and not also to the surrounding skin areas. In some embodiments, the age spots are on the face, the upper body or chest area, the legs, the hands, or the arms. In some embodiments, the cosmetic or pharmaceutical formulation comprises an extract from *Paeonia suffruticosa* root and *Ribes nigrum* seed oil.

Furthermore, the clinical advantages of using cosmetic and similar formulations made from the lipophilic active carrier compositions of the present invention over formulations containing i) only a vegetable oil, ii) a vegetable oil with a solubilization system but without the lipophilic bioactive extract or iii) a simple placebo representing the formulation base are shown herein.

The present invention provides a lipophilic carrier composition comprising *Cnidium monnieri* fruit extract, *Echium plantagineum* seed oil, ethanol, octyldodecanol, octyldodecyl oleate, and octyldodecyl stearoyl stearate. The present invention also provides a lipophilic carrier composition comprising *Paeonia suffruticosa* root extract, *Ribes nigrum* seed oil, ethanol, octyldodecanol, octyldodecyl oleate, and octyldodecyl stearoyl stearate.

### DETAILED DESCRIPTION

The term "topical" as used herein refers to the route of administration of a cosmetic composition that involves direct application to the body part being treated, e.g., the skin, hair or nails. Examples of topical application include application to the skin of creams, lotions, gels, ointments or other semisolids to rub-on, solutions to spray, or liquids to be applied by an applicator. Rinse-off applications with washes, cleansers, or shampoos are also examples of topical application. Typically, areas of the body suitable for application of the cosmetic compositions include the skin of the face, throat, neck, scalp, chest, back, ears, hands, arms, and other skin sites where dermatological conditions may occur.

The term "cosmetic" is intended to encompass compositions that improve the health and/or appearance of skin and hair and is used interchangeably with dermatologic and naturopathic, cosmeceutical, pharmaceutical, nutraceutical and other similar term.

As used herein, "root temperature" refers to a temperature of about 18-25°C, preferably 20-22°C.

As used herein, "homogeneous composition" refers to a composition that is a single phase that appears clear or translucent by visual inspection.

In the various embodiments discussed herein the vegetable oil is preferably one with beneficial pharmacological, cosmetic or dermatological properties. Most preferably the vegetable oil has a high level of polyunsaturated fatty acids, e.g., at least about 25%, at least about 40%, at least about 50%, at least about 60%, at least about 70%, at least about 80%, at least about 90%, or at least about 95%.

Vegetable oils which can be used in the present invention include any cosmetically compatible vegetable oil derived from a botanical source, such as a plant. Various parts of a plant may be used to obtain the vegetable oil, e.g., leaves, stems, bark, flowers, seeds, fruits, spores or roots. The vegetable oil may be obtained by conventional methods, e.g., by cold-press extraction and the like. Preferably the vegetable oil has beneficial pharmaceutical, cosmetic, or dermatological properties. Vegetable oils that include a significant proportion of polyunsaturated oils, such as the essential fatty acids omega-3 and omega-6, as well as omega-5 and omega-9 fatty acids, are particularly suitable for use in the inventive compositions and methods. In certain embodiments, the omega-3 fatty acids comprise alpha-linolenic acid (ALA) and its longer chain derivatives eicosapentaenoic acid (EPA) and docosahexaenoic acid (DHA) and the omega-6 fatty acids comprise linoleic acid (LA) and its longer chain derivatives such as gamma-linolenic acid (GLA) and arachidonic acid (AA). Topical application of some polyunsaturated fatty acids have been shown to have bioactivity, e.g., to improve the structure and function of cell membranes and improve skin barrier function. Improving skin barrier function reduces transepidermal water loss, leaving skin more hydrated, moisturized, and protected. The amount of vegetable oil used in the inventive compositions described herein is not particularly limited. A formulations scientist will readily be able to determine the appropriate amount of vegetable oil in the composition to achieve the desired properties in the composition. Typical embodiments of the invention include lipophilic carrier compositions comprising a vegetable oil in an amount from about 10-50%.

Vegetable oils that are well suited for the invention include *Ribes nigrum* (black currant) seed oil, *Echium planlagineum* (purple viper's bugloss) seed oil, baobab seed oil, black cumin seed oil, borage oil, burit fruit oil, calophyllum oil, elderberry seed oil, evening primrose oil, flax seed oil, gevuina nut oil, goji seed oil, hemp seed oil, jobs tears seed oil, jojoba oil, kiwi seed oil, neem oil, olive oil, passion fruit oil, pitanga seed oil (orange, red and purple varieties), pumpkin seed oil, raspberry seed oil, rose hip oil, sacha inch seed oil, safflower oil, sea buckthorn seed oil, sesame oil, soybean oil, sunflower seed oil and walnut oil. Vegetable oils particularly suited for use in the invention include *Ribes nigrum* (black currant) seed oil, *Echium plantagineum* (Purple Viper's Bugloss) seed oil or a combination thereof Suitable vegetable oils for use in the present invention include those in the following table.

**Table 1**

| Name of oil | Unsaturated FA (%) | Polyunsaturated FA (%) | Major constituents | Color | Origin |
|---|---|---|---|---|---|
| Apricot Kernel Oil | 95% | 29% | Oleic, linoleic, palmitic | clear to light yellow | Middle East |
| Baobab Seed Oil | 70% | 35% | Oleic, linoleic, palmitic, stearic | golden yellow | Southern Africa |
| Blackcurrant Seed Oil | 87% | 76% | Linoleic, a-linolenic, g-linolenic, oleic, palmitic | Clear yellow | Eastern Europe |
| Black Cumin Seed Oil | 78% | 58% | Linoleic, oleic, margaric, cis-11,14-eicosadienoic acid, stearic | yellow to amber | Middle East |
| Borage Oil | 85% | 69% | Oleic, linoleic, g-linolenic, palmitic | Dark green | North America and Eastern Africa |
| Burit Fruit Oil | 82% | 3% | Oleic, palmitic, linoleic, linolenic, stearic, | Red-Orange | Brazil |
| Calophyllum Oil | 71% | 31% | Oleic, linoleic, stearic, palmitic | clear green | Pacific Islands |
| Elderberry Seed Oil | 94% | 80% | Linoleic, a-linolenic, g-linolenic oleic, erucic, palmitic, stearic | deep dark green | North America |
| Evening Primrose Oil | 92% | 81% | Oleic, linoleic, g-linolenic, palmitic | yellow | Northern Asia |
| Flax Seed Oil | 91% | 72% | Linoleic, a-linolenic, oleic, palmitic | light yellow | South America |
| Gevuina Nut Oil | 82% | 32% | Oelic, palmitoleic, linoleic, a-linolenic | light yellow | Chile |
| Goji Seed Oil | 90% | 71% | Linoleic, Oleic, linolenic | Orange-yellow | Asia |
| Hemp Seed Oil | 92% | 80% | Linoleic, a-linolenic, palmitic, g-linolenic, stereadonic | Olive green | Northern Asia |
| Jobs Tears Seed Oil | 86% | 36% | Oleic, linoleic, palmitic | NA | Asia |
| Jojoba Oil | 90% | 6% | Gadoleic, erucic, oleic, linoleic, lignoceric, palmitic | pale yellow | Middle East & South America |
| Kiwi Seed Oil | 91% | 76% | a-Linolenic, linoleic, oleic, stearic, palmitic | yellow to amber | New Zeland |
| Neem Oil | 64% | 18% | Linoleic, oleic, stearic, palmitic | browny | Central Asia |
| Olive Oil | 84% | 8% | Oleic, Palmitic, linoleic | Light to medium green | Western Europe and Middle East |
| Passion Fruit Oil | 90% | 78% | Linoleic, oleic, palmitic, stearic, a-linolenic | Light | South America, |
| Pitanga Seeds (orange) Oil | 61% | 47% | Palmitic, linoleic, a-linoleic, oleic | NA | South America, Southeast Asia, and Australia. |
| Pitanga Seeds (red) Oil | 52% | 45% | Linoleic, palmitic, oleic, a-linoleic, palmitoleic | NA | South America, Southeast Asia, and Australia. |
| Pitanga Seeds (purple) Oil | 61% | 47% | Linoleic, palmitic, oleic, a-linoleic, palmitoleic | NA | South America Southeast Asia, and Australia. |
| Pumpkin Seed Oil | 84% | 57% | Linoleic, oleic, palmitic, stearic | dark green to dark red | Syria, Austria and Slovenia |
| Raspberry Seed Oil | 96% | 84% | Linoleic, a-linolenic, oleic | gold to reddish *color* | North America |
| Rose Hip Oil | 94% | 79% | Linolenic, linoleic, oleic, palmitic. | Amber | South America |
| Sacha inchi seed oil | 94% | 85% | a-Linoleic, linoleic, Oleic, palmitic, stearic | intense yellow to golden amber | Amazon river |
| Safflower Oil | 88% | 75% | Linoleic, oleic, palmitic, | light yellow | South Amercia |
| Sea buckthorn seed oil | 83% | 67% | Linoleic, a-linolenic, oleic, palmitic, stearic, vaccenic | golden | Europe and Asia |
| Sesame Oil | 87% | 45% | Oleic, linoleic, palmitic, stearic | Light yellow | Eastern Africa & Southern Africa |
| Soybean oil | 83% | 57% | Linoleic, oleic, a-linolenic, palmitic | NA | USA, Brazil, China |
| Sunflower Seed Oil | 77% | 66% | Linoleic, oleic, palmitic, stearic | slightly amber | North America |
| Walnut Oil | 84% | 56% | Linoleic, oleic, palmitic | light green | Eastern Europe. |

Lipophilic bioactive botanical extracts for use in the present invention are not particularly limited and include any lipophilic extract derived from a botanical source that has beneficial effects on the skin. The term botanical, as used herein, is intended to include material derived from organisms such as plants as well as fungi, algae, marine plant organisms, microorganism fermentation broths and other biological sources of cosmetic ingredients. Examples of various plant tissues include, but are not limited to whole plants, leaves, bark, roots, root bark, fruits, flowers, seeds, and pollen. The lipophilic bioactive botanical extract or compounds therefrom may be obtained by methods known in the art, e.g., by extraction with organic solvents, e.g., lipophilic organic solvents, or combinations of water and organic solvents, or by supercritical fluid carbon dioxide (SCF-CO₂) extraction with, or without, the addition or the presence of water.

Lipophilic bioactive botanical extracts include those having beneficial pharmaceutical, cosmetic, or dermatological properties. However, hydrophobic powders, waxes and other extracts having physico-chemical properties that require an inconvenient amount of cosmetically acceptable solvent, inconvenient amount of processing time, or elevated temperatures in order to incorporate the extract into a cosmetic formulation are particularly well suited for use in the inventive compositions and methods. Suitable lipophilic bioactive botanical extracts include those antioxidants listed in Table 2 that provide, in addition to antioxidant activity, a health benefit such as the health benefits listed in Table 2 under "Health Applications." Thus, the categories of lipophilic bioactive botanical extracts and antioxidants are not intended to be mutually exclusive. Typical embodiments of the invention include lipophilic carrier compositions comprising a lipophilic bioactive botanical extract or extracts in an amount from about 0.1-5%, about 0.3-4%, about 0.5-3%, about 0.7-3%, or about 1-2% (w/w). In other embodiments, the lipophilic carrier composition comprises a lipophilic bioactive botanical extract in an amount from about 0.1-0.3%, about 0.3-0.5%, about 0.5-0.7%, about 0.7-1%, about 1.1-1.3%, about 1.3-1.5%, about 1.5-1.7%, about 1.7-2%, about 2.1-2.3%, about 2.3-2.5%, about 2.5-2.7%, about 2.7-3%, about 3.1-3.3%, about 3.3-3.5%, about 3.5-3.7%, about 3.7-4%, about 4.1-4.3%, about 4.3-4.5%, about 4.5-4.7%, or about 4.7-5% (w/w).

Lipophilic bioactive botanical extracts that are particularly well suited to this invention are *Cnidium monnieri* fruit extract (enriched in the compound osthol) and *Paeonia suffruticosa* root extract (enriched in the compound paeonol) or a combination thereof. Use of these extracts has been found to protect against erythema and/or skin barrier function loss due to exposure of the skin to radiation or chemical stress.

Suitable antioxidants for use in the present invention are not particularly limited and may be antioxidants or free radical scavengers such as vitamins, synthetic antioxidants, or plant-derived antioxidants that protect at least the vegetable oil from endogenous oxidation and/or oxidation induced or accelerated by heat, radiation or the addition of pro-oxidant compounds, thus extending shelf life and expanding compatibility of bioactive ingredients in the inventive compositions and formulations that include the inventive compositions. A list of suitable antioxidants appears in Table 2 below.

**Table 2 - Liposoluble antioxidant actives and lipophilic bioactive botanical compounds**

| **Antioxidant/ lipophilic bioactive botanical compound** | **Molecular family** | **Source(s)** | **Health Applications** | **Other Applications** | **Ref.** |
|---|---|---|---|---|---|
| Ascorbyl-palmitate | Ester formed from ascorbic acid and palmitic acid | Synthesis | Immune booster; joint protection; promotes healing, stimulates collagen synthesis | To increase the shelf life of vegetable oils and potato chips | Cort, Antioxidant activity of tocopherols, ascorbyl palmitate, and ascorbic acid and their mode of action. J Am Oil Chem Soc. 1974 Jul:51(7):321-5. |
| Apigenin | Biflavone | Ampelopsis grossedentata stems; Allium sativum; Coriander fruit; Rosemary | Antioxidants, radical scavenger, anti-inflammatory, carbohydrate metabolism promoter, immunity system modulater. Induces autophagia; potent inhibitor of CYP2C9; monoamine transporter activator | Topical anti-inflammatory; substitute for corticoid therapy | Arsic et al, Preparation of novel apigenin-enriched, liposomal and non-liposomal, antiinflammatory topical formulations as substitute for corticosteroid therapy. Phytother Res. 2011 Feb;25(2):228-33. |
| Baicalein | Flavone | Roots of Scutellaria baicalensis & Oroxylum indicum | Cardiovascular protective; lowers blood pressure; antithrombotic, antiproliferative and anti-mitogenic; antioxidant; prostaglandin antagonis; free radical scavenger; eNOS inhibition: ICAM expression ↓ | Atopic dermatitis | Yun et al, Therapeutic effects of Baicalein on atopic dermatitis-like skin lesions of NC/Nga mice induced by dermatophagoides pteronyssinus. Int Immunopharmacol. 2010 |
| | | | | | Sep;10(9):1142-8 Srinivas NR, Baicalin, an emerging multi-therapeutic agent: pharmacodynamics, pharmacokinetics, and considerations from drug development perspectives. Xenobiotica. 2010 May;40(5):357-67 |
| Betulinic acid | Pentacyclic triterpenoid | Bark of several species of plants, principally the white birch; also present in rosemary extracts | Anti-retroviral, anti-malarial, and anti-inflammatory properties; potential anticancer agent, by inhibition of topoisomerase | To protect foods against oxidative rancidity. | Bracco et al, Production and use of natural antioxidants. Journal of the American Oil Chemists' Society 1981, 58 (6): 686-690 |
| Biochanin A | Phenolic (isoflavone) | Ganoderma lucidum, red | Potent ligands of the human aryl | Skin photoprotection | Moon et al, Dietary flavonoids: effects on |
| | | clover, soy, alfalfa sprouts, peanuts, chickpea and in other legumes. | hydrocarbon receptor; inhibits CYP19 expression and aromatase activity; anti-inflammatory; ↓NfkB translocation; chemopreventive; | | xenobiotic and carcinogen metabolism. Toxicol In Vitro. 2006 Mar;20(2):187-210. Lin et al, Topical isoflavones provide effective photoprotection to skin. Photodermatol Photoimmunol Photomed. 2008 Apr;24(2):61-6. |
| Bixin | Carotenoid | Seeds of the fruit of Achiote (Bixa orellana) tree | NA | To protect unsaturated lipids from oxidation; also used as a natural colorant by the food industry | Castro et al, The effects of colorifico on lipid oxidation, colour and vitamin E in raw and grilled chicken patties during frozen storage. Food Chemistry 2011, 124 (1): 126-131 |
| Boldine | Alkaloid | Boldo tree & Lindera aggregata | Antioxidant, cytoprotective, antitumour promoting, anti-inflammatory, anti-diabetic, anti-atherogenic actions, vasorelaxing, immunomadulator | Skin lightening agent. Improves the oxidative stability of bullfrog oil | O'Brien et al, Boldine and its antioxidant or health-promotion properties. Chem Biol Interact. 2006 Jan 5;159(1):1-17. |
| | | | | | Méndez et al, Fatty acid composition, extraction, fractionation, and stabilization of bullfrog (Rana catesbeiana) oil. Journal of the American Oil Chemists' Society (1998) 75(1): 79-83 |
| Caffeic acid | Hydroxycinna mic acid | Burdock, hawthorn, artichoke, pear, basil, thyme, oregano, apple, coffee beans | Anti-inflammatory and anti-cancer, UV-protection; lipoxygenase inhibitor | To reduce oxidation of fatty fish (mackerel) during storage | Eymard et al, Assessment of washing with antioxidant on the oxidative stability of fatty, fish mince during processing and storage. J Agric Food Chem. 2010, 58(10):6182-9. |
| Carnosic Acid & carnosal | Phenolic diterpene | Rosemary & Sage leaves | Antioxidant; inhibits lipid peroxidation; protects from carcinogens | Protects skin cells against UV-induced erythema Used as a preservative (anti-microbial) or antioxidant in food and nonfood products (e.g. toothpaste, mouthwash and chewing gum) | Reuter et al, Sage extract rich in phenolic diterpenes inhibits ultraviolet-induced erythema in vivo. Planta Med. 2007 |
| | | | | | Sep;73(11):1190-1 Weckesser et al, Screening of plant extracts for antimicrobial activity against bacteria and yeasts with dermatotogical relevance. |
| | | | | | Phytomedicine. 2007 Aug:14(7-8):508-16 G. Collins & Charles, Antimicrobial activity of Carnosol and Ursolic acid. Food Microbiology (1987) 4(4):311-315 |
| b-Carotene | Carotenoid | Sweet potatoes, carrots, goji, melon (cantaloupe), mangoes,apricots, Persimmon, spinach, kale, and many others | Anti-oxidant; anti-cancer; supports eye health and immune functions; protection from free radicals; cardio protection; bone health | Protection of skin from ozone-induced inflammation; protection of skin from infrared-induced free radicals; treatment of melasma; food coloring | Krinsky & Johnson, Carotenoids actions and their relation to health and disease. Mol Aspects Med. 2005 Dec;26(6):459-516. |
| | | | | | Valacchi et al, Beta-carotene prevents ozone-induced proinflammatory markers in murine skin. Toxicol Ind Health. 2009 May-Jun;25(4-5):241-7. |
| Crocetin | Carotenoid dicarboxylic acid | Crocus flower and Gardenia jasminoides fruits, and pistil of saffron | Improves quality of sleep; energy booster; neuroprotective; anti-tumor | To improve the oxidative stability of egg yolks of hens (fed with saffron); also used as a natural colorant | Botsoglou et al, Effect of dietary saffron (Crocus sativus L.) on the oxidative stability of egg yolk. Br Poult lot Sci. 2005 Dec;46(6):701-7. |
| Cryptoxanthin | Carotenoid (xanthophyll) | Petals & flowers of plants in the genus Physalis, orange rind, papaya, egg yolk, butter, apples, squash, pumpkins, tangerine | Provitamin A; Anti-oxidant; Free radical scavenger; helps repair DNA; chemopreventive; anti-cancer; benefits rheumatoid arthritis | Associated with butter skin hydration (in men only) | Namitha & Negi Chemistry and biotechnology of carotenoids Crit Rev Food Sci Nutr. 2010 Sep;50(8):728-60 Boelsma et al. Human skin condition and its associations with nutrient concentrations in serum and diet. Am J Clin Nutr.2003 Feb;77(2):348-55. |
| Curcumin | Phenolic | Curcuma longa (Turmeric) | Anti-tumor, anti-inflammatory & anti-oxidant, chemopreventive & chemotherapeutic; inhibits iNOS, COX & LOX, induces HO-1; epigenetic agent | To improve transdermal delivery Acne, skin aging; psoriasis | Rungphanichkul et al, Preparation of curcuminoid niosomes for enhancement of skin permeation. Pharmazie. 2011 Aug;66(8):570-5. Lima et al, Curcumin induces heme oxygenase-1 in normal human skin fibroblasts through redox signaling: relevance for anti-aging intervention. Mol Nutr Food Res. 2011 Mar;55(3):430-42 |
| | | | | | Jurenka, Anti-inflammatory properties of curcumin, a major constituent of Curcuma longa: a review of preclinical and clinical research. Altern Med Rev. 2009 Jun; 14(2):141-53. Review |
| Emodin | Anthraquinone | Himalayan rhubarb & buckthorn & Aloe vera leaf, Frangula bark | Anti-inflammatory, anti-tumor, antipsychotic, profibrinolytic & wound healing; inhibits NFkB activation & ICAM expression; CK2 inhibitor; anti-herpes | NA | El-Shemy et al, Antitumor properties and modulation of antioxidant enzymes' activity by Aloe vera leaf active principles isolated via supercritical carbon dioxide extraction. Curr Med Chem. 2010; 17(2):129-38. Review |
| Epigallocatechin gallate | Flavanol | Mainly from green tea | Anti-tumor, anti-HIV, anti-inflammatory, weight control | To protect liposomes against oxidative rancidity | Huang & Frankel, Antioxidant Activity of Tea Catechins in Different Lipid Systems, Journal of Agricultural and Food Chemistry 1997, 45 (8): 3033-3038 |
| Ferulic acid | Hydroxycinna mic acid | seeds of coffee, apple, artichoke, peanut, and orange; seeds and cell walls of commelinid plants (rice, wheat, oats, and pineapple) | Anti-tumor, bone health, cardiovascular health, UV protection | Most effective in protecting linoleic acid from autooxidation | Kikuzaki et al, Antioxidant properties of ferulic acid and its related compounds. J Agric Food Chem. 2002, 50(7): 2161-8. |
| Honokiol & Magnetol | Biphenyl neolignans | Cones, bark, and leaves of Magnolia grandifloris | Antioxidative, anti-inflammatory, anti-tumor, anti-angiogenic, anti-diabetic, anti-microbial, antifungal, antifungal, anti-neurodegeneration, anti-depressant, pain control- hormonal regulatian, regulation, cardiovascular and liver protective properties ; Modulation of the NFkB signaling pathway | Reduces UVB-induced inflammation & skin cancer; Anti-acne; anti-aging skin cream | Park et al, In vitro antibacterial and anti-inflammatory effects of honokiol and magnolol against Propionibacterium sp. Eur J Pharmacol. 2004 Aug 2;496(1-3):189-95. |
| | | | | | Vaod et al, Honokiol, a phytochemical from the Magnolia plant, inhibits photocarcinogenesis by targeting UVB-induced inflammatory mediators and cell cycle regulators: development of topical formulation. Carcinogenesis. 2010 Nov;31(11):2004-11 Shen et al, Honokiol and magnolol as multifunctional antioxidative molecules for dermatolagic disorders. Molecules. 2010 Sep 16:15(9):6452-65 Lee et al, Therapeutic applications of compounds in the Magnolia family. Pharmacol Ther. 2011 May; 130(2):157-76 |
| Lutein | Carotenoid | Spinach, turnip greens, romaine lettuce, eggs, red pepper, pumpkin, mango, papaya, oranges, kiwi, peaches, squash, legumes, brassicates, prunes, sweet potatoes, honeydew melon, rhubarb, plum, avocado, pear | Macular degeneration, cataracts, visual performance | Protection of soybean oil from photo-oxidation | Lee & Mi, Effects, quenching mechanisms, and kinetics of carotenoids in chlorophyll-sensitized photooxidation of soybean oil.. Agric. Food Chem., 1990, 38 (8): 1630-1634 |
| Luteolin | Biflavonoid | Reseda luteola, Achillea millefolium, Chamomillae requtita, Cynara scolymus, Thymus vulgaris, Erigeron canadensis, Propolis,etc_{.} | Strong antioxidant and radical scavenger, anti-cancer; fights allergies, anti-inflammatory; cataract; competitive inhibitor of xanthine oxidase; inhibits lipid peroxidation | Topical anti-inflammatory; anti-pruritus | Baolin et al, Topical application of luteolin inhibits scratching behavior associated with allergic cutaneous reaction in micee. planta Med. 2005 May;71(5):424-8. |
| Lycopene | Carotenoid | Vietnam Gac. tomatoes, grapefruit, watermelon, guava, apricots, carrots, autumn olive | Anti-cancer, neuroprotective, cardioprotection, male fertility | Most powerful carotenoid quencher of singlet oxygen; used to preserve. oils | Montesano et al , Pure lycopene from tomato preserves extra virgin olive oil from natural oxidative events during storage. J Agric Food Chem, 2006, 83(11): 933-941 |
| Moronic acid | Triterpenic acid | Rhus javanica (a sumac), mistletoe, frankincense, olibanum | Anti-HIV activity; also active against herpes simplex virus 1 | NA | Donglei et al, Anti-AIDS Agents 69. Moronic Acid and Other Triterpene Derivatives as Novel Potent Anti-HIV Agents. Journal of Medicinal Chemistry 2006.49 (18): 5462-9. |
| Oleanolic acid | Triterpenic acid | Olive leaf, american pokeweed, honey mesquite, garlic, java apple, cloves, and many other Syzygium species. | Anti-inflammatory, chemoprevention, anti angiogenic | For protection of vegetable oils | Guinda et al, Supplementation of oils with oleanolic acid from the olive leaf (olea europaea), European Journal of Lipid Science and Technology 106 (1): 22-26,2004 |
| Paprika (capsaicin) | Phenolic acid | Fruits of Capsicum annuum (chili peppers), | Anti-inflammatory and analgesic; animal repellent; antibacterial | To increase oil stability during frying | Yang http://ww.springerli nk.com/content/?Aut hor=Cheut-Young-1-Yang et al, Capsaicin and tocopherol in red pepper seed oil enhances the thermal oxidative stability during frying, Journal of Food Science and Technology, 2010, 47(2): 162-165 |
| Phytoene | Carotenoid (colorless) | In most fruits and vegetables; also in algae (Dunatiella) | UV absorber, anti-inflammatory, DNA & collagen protection, anti-pigmentation | Sunscreen stabilizer | L. von Oppen-Bezalel (2009). "Lightening, Boosting and Protecting with Colorless Carotenoids". Cosmetics & Toiletries magazine 124(3): 66-75. |
| Phytofluene | Carotenoid (colorless) | Tomatoes and other fruits & vegetables; also in algae (Dunaliella) | UV absorber, anti-inflammatory, DNA & collagen protection, anti-pigmentation | Sunscreen stabilizer | L. von Oppen-Bezalel (2007). "UVA, A Main Concern in Sun Damage: Protection from the Inside and Outside with Phytoene, Phytofluene, the Colorless Carotenoids and more". SÖFW-Journal. |
| Quercetin | Flavonol | Citrus fruits, apples, onions, parsley, sage, tea, and red wine, olive oil, grapes, dark cherries, and dark berries | Antihistamine and anti-inflammatory; may help protect against heart disease and cancer. | To reduce the oxidative delerioration of cottonseed o/w emulsions | Kiokias et al. In Vitro Activity of Vitamins, Flavonoids, and Natural Phenolic Antioxidants Against the Oxidative Deterioration of Oil-Based Systems. Crit Rev Food Sci Nutr. 2008, 48(1): 78-93 |
| Reticuline | Alkaloid | Lindera aggregate; roots of Po lyalthia cerasoides | Vasorelaxant | Hair growth | Nakaoji et al, Norreticuline and reticuline as possible new agents for hair growth acceleration. Biol Pharm Bull. 1997 May;20(5):586-8. |
| Rosmarinic acid | Phenolic acid | Found in many plants, including rosemary, basil, | Antiviral, antibacterial, anti-inflammatory, | To slow rancidity in food, fats, and | Frankel et al, Antioxidant/ Activity of a Rosemary |
| | | peppermint, lemon balm (Melissa officinalis), and fennel. | hepatoprotective, antispasmodic | oils. | Extract and Its Constituents, Carnosic Acid, Carnosol, and Rosmarinic Acid, in Bulk Oil and Oil-in-Water Emulsion, J. Agric. Food Chem., 1996, 44 (1); 13l-135 |
| Salvigenin | Flavone | Rosmarinus officinalis leaves & Salvia leriaefolia | Anti-oxidant, anti-inflammatory, anti-plasmodial | Topical anti-inflammatory | Kuo et al, Anti. inflammatory effects of supercritical carbon dioxide extract from Rosmarinus offcinalis leaves. J Agric Food Chem. 2011 Apr 27;59(8):3674-85 |
| Tanshinone IIA | Diterpenoid naphthoquinon e | Found in Salvia miltiorrhiza & Aquilaria sinensis; part of the Chinese medicine Tanshen | Anti-oxidant, anti-inflammatory, Inhibits AP-1 activity; antagonist of PPARγ; anti obesity; anti psoriasis; anti angiogenic; improves circulation; cardioprotective | NA | Li et al, Tanshinone IIA Inhibits Growth of Keratinocytes through Cell Cycle Arrest and Apoptosis: Underlying Treatment Mechanism of Psoriasis. Evid Based Complement Alternat Med. 2012;2012:927658. |
| Thymoquinone | Quinone | Nigella sativa (cumin) & Thymus vulgaris L. aerial flowering parts & Satureja montana | anti-cancer, analgesic anticonvulsant, angiogenesis inhibitor; histone cleacetylase inhibitor; strong anti-oxidant: protects against renal injury; analgesic | Inhibits corneal angiogenesis | Ragheb et al, The protective effect of thymoquinone, an antioxidant and anti-inflammatory agent, against renal injuiy: a review. Saudi J Kidney Dis Transpl. 2009 Sep;20(5):741-52. Review. |
| Ursolic acid | Pentacyclic triterpene acid | Apples, basil, bilberries, cranberries, elder | Cardioprotective, anticancer, cytotoxic, antitumor, | Anti-wrinkle | Sultana N., Clinically useful anticancer, antitumor, and |
| | | flower, peppermint, rosemary, lavender, oregano, thyme, hawthorn, prunes | antioxidant, anti-inflammatory, anti-HIV, acetyl cholinesterase, α-glucosidase, antimicrobial, and hepatoprotective | | antiwrinkle agent, ursolic acid and related derivatives as medicinally important natural product. J Enzyme Inhib Med Chem. 201126(5):61642 |
| Vanillic acid | Phenolic acid | Angelica sinensis, Origanum vulgare, Allium cepa, Allium sativum, Annoracia rusticana, & Aspalathus linearis | Anti-oxidant; anti-diabetic; hepatoprotective | inhibition of alpha-metanocyte-stimulating hormone-stimulated melanogenesi s; Preservation of freslt food | Chou et al, Antioxidative characteristics and inhibition of alpha-melanacyte-stimulating hormone-stimu lated melanogenesis of vanillin and vanillic acid from Origanum vulgare. Exp Dermatal. 2010 Aug; 19(8):742-50. |
| Verbascoside (acteoside) | Phenylethanoi d glycoside | Buddleja davidii cell cultures | antioxidant, anti-inflammatory, photoprotective and chelating,antiprolifer ative | Promotes skin repair and ameliorates skin inflammation; ↑GST activity | Vertuani et al, Activity and stability studies of verbascoside, a novel antioxidant, in dermo-cosmetic and pharmaceutical topical formulations. Molecules. 2011 Aug 18;16(8):7068-80 |
| Vitexin | Biflavonoid | Ampelopsis grossedentata stems; Jatropha curcas leaves; hawthorn herb; Arnebia hispidissima; Fenugreek; Ochrocarpus longifolius; Acer palmatum | Anti-oxydant, anti-tumor, anti-inflammatory, hypotensive, antispasmodique; anti-histamine activity | Inhibition of adipogenesis; Anti-glycation activity | Kim et al, Vitexin, orientin and other flavonoids from Spirodela polyrhiza inhibit adipogenesis in 3T3-L1 cells. Phytother Res. 2010 Oct;24(10):1543-8. Prabhakar et al, Pharmacological investigations on vitexin. Planta Med. 1981 Dec;43(4):396-403. |
| | | | | | Peng et al, Inhibitary effect of mung bean extract and its constituents vitexin and isovitexin on the formation of advanced glycation endproducts. Food Chemistry (2008) 106 (2): 475-481 1 |
| Vitamin E | Vitamin | Wheat germ oil, sunflower oil, nuts and nut oils, leafy vegetables, avocado, asparagus, kiwi, broccoli, pumpkin, mangoes, sweet potatoes, papayas | Anti-inflammatory, cardioprotective, anti-tumor | Anti-wrinkle, photoprotective, and skin barrier stabilizing properties To increase oil stability | Thiele & Ekanayake-Mudiyanselage, Vitamin E in human skin: organ-specific physiology and considerations for its use in dermatalogy. Mol Aspects Med. 2007 28(5-6):646-67. Kiokias et al, In Vitro Activity of Vitamins, Flavanoids, and Natural Phenolic Antioxidants Against the Oxidative Deterioration of Oil-Based Systems. Crit Rev Food Sci Nutr. 2008,48(1): 78-93 |
| Zeaxanthin (lutein isomer) | Carotenoid | Leaves of most green plants; also paprika, saffron | Anti-oxidant; eye health; macular degeneration; cataracts | Food dye UV protection for skin; inhibits skin lipid peroxidation | Lien et al, Nutritional influences on visual development and function. Prog Retin Eye Res. 2011 May;30(3):(88-203 Palombo et al, Beneficial long-temi effects of combined oral/topical antioxidant treatment with the carotenoids lutein and zeaxanthin on human skin: a double-blind, placebo-controlled study. Skin Pharmacol Physiol. 2007;20(4):199-210 )-199-210 |

When present, antioxidants may be present in an amount that increases the Rancimat induction time (according to the ISO Method no. 6886-2006) to greater than 3 hours, greater than 4 hours, or greater than 5 hours, or more. Natural antioxidants may be selected from botanical extracts known to have antioxidant activity. Typically, amounts from about 0.1-2% (w/w) of such extracts are sufficient to impart the desired stability to a lipophilic carrier composition. Preferably, amounts from 0.2% to 2.0% or from 0.5% to 1.5% (w/w) are present in the inventive lipophilic carrier compositions. Suitable antioxidants for use with the present invention include natural or synthetic caffeic acid and carnosic acid and mixtures thereof. Preferably, the antioxidants are *Rosmarinus officinalis* (rosemary) leaf extract and/or *Solidago virgaurea* (goldenrod) extract. Most preferable is a combination of *Rosmarinus officinalis* extract and *Solidago virgaurea* extract. When used in normal daily conditions, oils or any compounds used in formulations may be exposed to light (UV) or a combination on light, heat, and air. Furthermore, they may also be in contact with other compounds having pro-oxidative properties. In such conditions, various types of reactive oxygen species or free radicals may be generated. Antioxidants usually target specific types of reactive oxygen species or free radicals. To ensure broader protection (especially of the vegetable oil), rosemary and goldenrod extracts together may be suitable.

Nevertheless, rosemary extract and goldenrod extract may also be used separately. For example, either only 0.1 % of rosemary extract (and no solidago extract) or only 0.1% of solidago extract (and no rosemary extract) in the lipophilic carrier composition may be used.

The solubilizing system of the present invention preferably includes a cosmetically acceptable alcohol such as ethanol, and preferably also octyldodecanol and a fatty acid ester of octyldodecanol, e.g., octyldodecyl oleate and/or octyldodecyl stearoyl stearate. In some embodiments, the solubilization system comprises ethanol at 2-20% (w/w) of the total solubilization system, octyldodecanol at 20-40% (w/w) of the total solubilization system, a first fatty acid ester of octyldodecanol at 20-55% (w/w) of the total solubilization system, and, optionally, a second fatty acid ester of octyldodecanol at 20-55% (w/w) of the total solubilization system.

Preferably, the amounts of the components of the solubilization system are chosen such that, when the solubilization system is used to prepare a lipophilic carrier composition, the ethanol is present in an amount from 2-15%, 2-10%, 2-7%, 3-10%, or 4-8% (w/w) of the lipophilic carrier composition. Preferably, the octyldodecanol is present in the lipophilic carrier composition in an amount from 15-35%, 15-30%, 20-30%, or 25-30% (w/w) of the lipophilic carrier composition. Preferably, octyldodecyl oleate is present in an amount from 15-25%, 15-20%, or 20-25% (w/w) of the lipophilic carrier composition. Preferably, octyldodecyl stearoyl stearate is present in an amount from 5-15%, 5-10%, or 10-15% (w/w) of the lipophilic carrier composition.

In certain embodiments, the solubilization system may contain different components from those described above. In such embodiments, the solubilization system is such that the lipophilic bioactive botanical extract may be dissolved therein, if necessary by the use of elevated temperatures (e.g., 40-100°C), and remain dissolved as the temperature is lowered to about room temperature, at which point the vegetable oil is added. The solubilization system allows for the combining of bioactive botanical extract and vegetable oil in one composition, without unacceptable oxidation of the fatty acids in the vegetable oil.

Lipophilic bioactive botanical extracts can be solubilized in a lipophilic carrier composition comprising a vegetable oil using a solubilizing system as described above. In one embodiment, a first phase is prepared by combining the lipophilic bioactive botanical extract and the solubilizing system to form a mixture. The mixture is then heated to between 40°C and 100°C under a nitrogen atmosphere until complete solubilization of the extract, as judged by visual inspection. The solution is then cooled, preferably to a temperature of about 35-40°C, about 30-35°C, about 25-30°C, about 20-25°C, or about 15-20°C, the vegetable oil is added, and the mixture is cooled to room temperature, if not already at room temperature. A second phase is prepared separately by dissolving the antioxidant extracts in a cosmetically compatible alcohol, e.g., ethanol, and the resulting mixture is then agitated at room temperature until the extracts are dissolved. The first and second phases are then combined together under agitation until a homogenous mixture is obtained.

In certain embodiments, the lipophilic carrier composition of the present invention comprises more than one lipophilic bioactive botanical extract, more than one vegetable oil, or more than one antioxidant. In certain embodiments, the lipophilic carrier composition comprises two lipophilic bioactive botanical extracts, two vegetable oils, or two antioxidants.

**Table 3. Representative ranges of ingredients suitable for certain embodiments of lipophilic bioactive botanical extracts solubilized with vegetable oils (lipophilic carrier compositions). Percentages represent w/w of the total lipophilic carrier composition.**

| | |
|---|---|
| Vegetable Oil | 20-40% |
| Octyldodecanol | 15-35% |
| Octyldodecyl Oleate, | 15-25% |
| Octyldodecyl Stearoyl Stearate | 5-15% |
| Lipophilic bioactive botanical extract | 0.1-5.0% |
| Ethanol | 2-15% |
| Antioxidant | 0.01-2.0% |

In certain embodiments, the vegetable oil is present at 20-25%, 25-30%, 30-35%, or 35-40% (w/w). In certain embodiments, the octyldodecanol is present at 15-20%, 20-25%, 25-30%, or 30-35% (w/w). In certain embodiments, the octyldodecyl oleate is present at 15-20% or 20-25% (w/w). In certain embodiments, the octyldodecyl stearoyl stearate is present at 5-10% or 10-15% (w/w). In certain embodiments, the lipophilic bioactive botanical extract is present at 0.1-0.5%, 0-5-1%, 1-2%, 2-3%, 3-4%, or 4-5% (w/w).

The presolubilized extracts in lipophilic carrier compositions are easily incorporated into a wide variety of product types that include but are not limited to solid and liquid compositions intended for topical use such as lotions, creams, gels, sticks, sprays, shaving creams, ointments, cleansing liquid washes and solid bars, pastes, powders, mousses, masks, peels, makeup, and wipes. The compositions may be used in conjunction with other devices such as skin abrading, skin massaging, or electro-stimulation devices, light-therapy devices, ultrasound devices, radio frequency devices, thermal/cooling devices, iontophoresis devices, and micro-penetration devices.

### EXAMPLES

To assess the efficacy of skin protective, soothing and repairing effects as well as the oxidation stability of a cosmetic topical product incorporating the inventive lipophilic carrier compositions, the following generic formulations were prepared, In a first embodiment, a lipophilic bioactive botanical extract of *Cnidium monnieri* fruit in a lipophilic carrier composition that included *Echium plantaginium* seed oil was used to prepare a cosmetic formulation that was evaluated for skin protective, soothing and repairing effect upon exposure to a chemical agent.

In a second embodiment, a lipophilic bioactive botanical extract of *Cnidium monnieri* fruit in a lipophilic carrier composition that included *Echium plantaginium* seed oil was used to prepare a cosmetic formulation that was evaluated for skin protective, soothing and repairing effect upon exposure to ultraviolet radiation.

The following general formulations were prepared in order to assess the stability and efficacy of the inventive compositions:

**Table 4: Formulations**

| **Ingredients** | | **Formula 1** | **Formula 2** | **Formula 3** | **Formula 4** |
|---|---|---|---|---|---|
| Total | | 100.00 | 100.00 | 100.00 | 100.00 |

| **INCI Name** | **Trade Name** | | | | |
|---|---|---|---|---|---|
| **Water Phase (Phase A):** | | | | | |
| Water | | 96 | 95.3 | 95.3 | 95.3 |
| Sodium Polyacrylate | Cosmedia SP | 0.6 | 0.6 | 0.6 | 0.6 |
| Preservative | Euxil K300 | 0.4 | 0.4 | 0.4 | 0.4 |

| **Oil Phase (Phase B):** | | | | | |
|---|---|---|---|---|---|
| Cetearyl Alcohol | Hydrenol D | 2 | 2 | 2 | 2 |
| Ceteareth-20 | Eumulgin B2 | 1 | 1 | 1 | 1 |

| **Active (Part C):** | | | | | |
|---|---|---|---|---|---|
| **Part C1** | | | | | |
| Lipophilic bioactive extract‡ | | 0 | 0 | 0 | 0.01 |
| Octyldodecanol | | 0 | 0 | 0.63 | 0.62 |
| Octyldodecyl Oleate | | 0 | 0 | 0.39 | 0.39 |
| Octyldodecyl Stearoyl Stearate | | 0 | 0 | 0.18 | 0.18 |

| **Part C2** | | | | | |
|---|---|---|---|---|---|
| Vegetable Oil† | | 0 | 0.7 | 0.7 | 0.7 |
| Rosemary leaf extract | | 0 | 0 | 0.002 | 0.002 |
| Goldenrod Extract Ethanol | | 0 | 0 | 0.002 | 0.002 |
| | | 0 | 0 | 0.096 | 0.096 |
| Total Oil Loading: | | 3.00 | 3.70 | 3.70 | 3.70 |
| Total Water Loading: | | 97.00 | 96.30 | 96.30 | 96.30 |

| | | | | | |
|---|---|---|---|---|---|
| Exemplified lipophilic bioactive botanical extract: Example 6: *Cnidium monnieri* Fruit Extract; Example 7: *Paeonia suffruticosa* Root extract. ^{†} Exemplified Vegetable Oil Example 6: *Echium plantagineum* seed oil; Example 7: *Ribes nigrum* (black currant) seed oil. | | | | | |

### COMPARATIVE EXAMPLE 1. METHOD OF PREPARING BASE CREAM FORMULA 1

Phase A and Phase B (see Table 4) were prepared separately by weighing their ingredients at room temperature, warming the ingredients to 75°C, and then combining the respective ingredients of the two phases with stirring. The phases were then warmed to about 75°C and combined with agitation until homogenous.

### COMPARATIVE EXAMPLE 2. METHOD OF PREPARING FORMULA 2, BASE CREAM PLUS VEGETABLE OIL

Phase A and Phase B were prepared separately by weighing their ingredients at room temperature, warming the ingredients to 75°C, and then combing the respective ingredients of the two phases with stirring. The phases were then warmed to about 75°C and combined with homogenization until homogenous. Vegetable oil was then added at 75°C with agitation until homogenous.

### COMPARATIVE EXAMPLE 3. METHOD OF PREPARING FORMULA 3, BASE CREAM, VEGETABLE OIL PLUS SOLUBILIZATION SYSTEM AND ANTIOXIDANTS

Phase A and Phase B were prepared separately by weighing their ingredients at room temperature, warming the ingredients to 75°C, and then combing the respective ingredients of the two phases with stirring. The phases were then warmed to about 75°C and combined with agitation until homogenous. Part C was prepared by separately combining the respective ingredients of Parts C1 (lipophilic bioactive extract is not included) and C2 with stirring and then combining Parts C1 and C2 at room temperature. Part C was then added to the mixture of Phase A and Phase B and homogenized.

### EXAMPLE 4. METHOD OF PREPARING FORMULA 4, ACTIVE CREAM INCORPORATING INVENTIVE PRESOLUBILIZED LIPOPHILIC BIOACTIVE BOTANICAL EXTRACT IN A LIPOPHILIC CARRIER COMPOSITION

A presolubilized lipophilic bioactive botanical extract was prepared as follows: Part C was prepared by first combining the lipophilic bioactive botanical extract in a mixture of octyldodecanol, octyldodecyl oleate, and octyldodecyl stearoyl stearate while heating to between 65-70°C under a nitrogen atmosphere until complete solubilization of the extract resulting in a clear or translucent solution. The solution was then cooled to 45°C, the vegetable oil was added, and the mixture was cooled to room temperature. A mixture of the antioxidants dissolved in ethanol was then added to the cooled solution with agitation. This presolubilized lipophilic bioactive botanical extract in a lipophilic carrier composition (Part C) was then combined with a base cream of Formula 1 (comparative example 1) and homogenized.

### EXAMPLE 5

### PROTECTIVE EFFECT OF ANTIOXIDANTS

Experiments were performed using the Rancimat method (Method: ISO 6886-2006) to assess the efficacy of a lipophilic carrier composition comprising *Rosmarinus officinalis* leaf extract and *Solidago virgaurea* (Goldenrod) extract to protect oil derived from *Echium plantagineum* seeds or from *Ribes nigrum* seeds from oxidation when the oil and the antioxidants are present in the lipophilic carrier composition. In the Rancimat method, a sample of oil is heated under atmospheric pressure, and air is allowed to bubble through the oil at a selected temperature. Under these conditions, a lipoperoxidative reaction occurs and the short-chain volatile acids produced thereby are recovered and measured conductometrically in distilled water. The time required to produce a sudden increase in conductivity, due to the formation of volatile acids, determines an induction time which can be defined as a measure of the oxidative stability of the oil. A Metrohm Rancimat model 743® (Herisau, Switzerland) was used. Results obtained showed that the presence of *Rosmarinus officinalis* leaf extract and *Solidago virgaurea* extract protected the oil derived from *Echium plantagineum* seeds (Table 5) or from *Ribes nigrum* seed extract (Table 6) from degradation induced by heat and air contact. Furthermore, it was demonstrated that the addition of other ingredients of the lipophilic carrier composition, e.g., a *Cnidium monnieri* fruit extract or a *Paeonia suffruticosa* root extract, does not negatively affect the stability of *Echium plantagineum* oil or *Ribes nigrum* oil towards oxidation.

**Table 5. Effect of various components of the Echium plantagineum seed oil-based compositions on Rancimat time.**

| *Echium plantagineuM* seed oil (%) | | Octyldodecanol (%) | octyldodecyloleate, octyldodecyl stearoylstearate (%) | Ethanol (%) | *Rosmarinus officinalis* (Rosemary) leaf extract (%) | *Solidago virgaurea* (Goldenrod) extract (%) | *Cnidiummonnieri* Fruit Extract (%) | Rancimat time (hr) |
|---|---|---|---|---|---|---|---|---|
| Source 1 | Source 2 | | | | | | | |
| 100 | - | - | - | - | - | - | - | 0.3 |
| - | 100 | - | - | - | - | - | - | 0.1 |
| 35 | - | 29.5 | 30 | 4.8 | - | - | - | 3.3 |
| 35 | - | 29.5 | 30 | 4.8 | 0.1 | 0.1 | - | 19.6 |
| 35 | - | 29.5 | 30 | 4.8 | 0.1 | 0.1 | 0.5 | 20.4 |

**Table 6. Effect of various components of the Ribes nigrum (black currant) seed oil-based compositions on Rancimat time.**

| *Ribesnigrum* (Black Currant) seed oil (%) | | Octyldodecanol (%) | octyldodecyloleate, octyldodecylstearoylstearate (%) | Ethanol (%) | *Rosmarinus officinalis* (Rosemary) leaf extract (%) | *Solidago virgaurea* (Goldenrod) extract (%) | *Paeonia suffruticos* a Root extract (%) | Rancimat time (hr) |
|---|---|---|---|---|---|---|---|---|
| Source 1 | Source 2 | | | | | | | |
| 100 | - | - | - | - | | - | - | 0.2 |
| - | 100 | - | - | - | - | - | - | 0.1 |
| 35 | - | 29 | 30 | 4.8 | - | - | - | 7.4 |
| 35 | - | 29 | 30 | 4.8 | 0.1 | 0.1 | - | 30.7 |
| 35 | - | 29 | 30 | 4.8 | 0.1 | 0.1 | 1.0 | 33.4 |

### SUMMARY OF CLINICAL TRIALS

Several lipophilic bioactive botanical extracts solubilized using the solubilization system described herein and present in a lipophilic carrier composition comprising vegetable oil were tested in human clinical trials for efficacy. These tests showed safety and efficacy in preventing and treating skin erythema and lessening the reduction of the skin barrier function after exposure to radiation and chemical stress.

### EXAMPLE 6: ECHIUM PLANTAGINIUM-BASED ACTIVE CREAM WITH CNIDIUM MONNIERI FRUIT EXTRACT

Formula 1-4 as described above were prepared, wherein the lipophilic bioactive botanical extract was *Cnidium monnieri* fruit extract and the vegetable oil was *Echium plantaginium* seed oil.

The skin protective, soothing and repairing effect of a cosmetic topical product incorporating the presolubilized *Cnidium monnieri* fruit extract in a lipophilic carrier composition comprising *Echium plantaginium* seed oil upon exposure of the skin to a chemical agent was evaluated by applying topical treatments (2 mg/cm²) of each of Formulas 1 through 4.

### EXAMPLE 6A SKIN PROTECTIVE EFFECT UPON EXPOSURE TO CHEMICAL AGENT

The test was carried out on a panel of 10 healthy volunteers. Selected skin areas were kept untreated and unexposed to the chemical agent as controls. Other skin areas were treated with the relevant formulation for a period of 10 days (from Day 1 to Day 10) preceding exposure to the chemical agent.

On Day 11, a solution of sodium lauryl sulfate (SLS) was applied to the skin using Finn chambers to chemically aggress the skin. Finn chambers were kept in contact with the skin for 20±4 hours. On Day 12, the Finn chambers were removed and skin erythema and the skin barrier function were assessed to measure the protective effect of the treatment with the formulations. The skin barrier function was assessed by the measurement of trans-epidermal water loss (TEWL) using the TEWAMETER 300^{®} (Courage+Khazaka, electronic GmbH) apparatus and skin erythema was measured using the MEXAMETER^{®} MX 18 (Courage+Khazaka, electronic GmbH) apparatus. Results are shown in Tables 7 and 8.

Application of the *Echium Plantaginium* Active Cream with *Cnidium Monnieri* Fruit Extract prior to the application of the SLS-containing Finn chambers resulted in a reduction of the SLS-induced increase in TEWL (Table 7). This demonstrated that the *Echium Plantaginium* Active Cream with *Cnidium Monnieri* Fruit Extract (made with a lipophilic carrier composition of the present invention) can protect the skin barrier function from a chemical aggression.

**Table 7. Effect of a topical composition incorporating a presolubilized Cnidium monnieri fruit extract in a lipophilic carrier composition comprising Echium plantagineum seed oil on the prevention of chemically-induced skin barrier damage - reduction of TEWL increase when cream formations produced with the lipophilic carrier composition are applied as a preventive treatment.**

| | Chemically-induced increase in TEWL (%)¹ |
|---|---|
| Control (untreated skin) | 400.5 |
| Formula 1: base cream | 350.6 |
| Formula 2: Formula 1 with *Echium plantagineum* seed oil | 319.3 (p=0.020) vs Placebo⁵ |
| | (p=0.011) vs Active cream⁶ |
| Formula 3: Formula 2 with antioxidants and solubilization system | 331.3 (p=0.028) vs Placebo³ |
| | (p=0.016) vs Active cream¹ |
| Active Cream Formula 4: Formula 3 with presolubilized *Cnidium monnieri* fruit extract | 289.9 (p<0.002) vs Placebo² |

| | |
|---|---|
| ¹Compared to non-chemically stressed skin (baseline) ²Statistically significant when compared to Placebo cream ³Statistically significant when compared to Placebo cream ⁴Statistically significant when compared to Active cream ⁵Statistically significant when compared to Placebo cream ⁶Statistically significant when compared to Active cream | |

The measurement of skin erythema (Table 8) also demonstrated that the application of the *Echium plantaginium* Active Cream with *Cnidium monnieri* Fruit Extract can protect from chemically-induced skin erythema.

The above results demonstrate that a topical composition comprising a lipophilic carrier composition of the present invention that was produced using a presolubilized *Cnidium monnieri* fruit extract provided better protection against an increase in TEWL than the same composition lacking certain ingredients present in the inventive composition.

**Table 8. Effect of a tropical composition incorporating a presolubilized Cnidium monnieri fruit extract in a lipophilic carrier composition comprising Echium plantagineum seed oil on chemical agent-induced skin erythema when applied as a cream formulation produced with the lipophilic carrier composition as a preventive treatment**

| | Chemically-induced increase in skin erythema (%)¹ |
|---|---|
| Control (untreated skin) | 59.0 |
| Formula 1: base cream | 51.2 |
| Formula 2: Formula 1 with *Echium plantagineum* seed oil | 43.8 (p=0.030) vs Placebo⁵ |
| | (p=0.004) vs Active cream⁶ |
| Formula 3: Formula 2 with antioxidants and solubilization system | 41.3 (p=0.002) vs Placebo³ |
| | (p=0.033) vs Active cream⁴ |
| Active Cream Formula 4: Formula 3 with presolubilized *Cnidium monnieri* fruit extract | 36.8 (p<0.001) vs Placebo² |

| | |
|---|---|
| ¹Compared to non-chemically stressed skin (baseline) ²Statistically significant when compared to Placebo cream ³Statistically significant when compared to Placebo cream ⁴Statistically significant when compared to Active cream ⁵Statistically significant when compared to Placebo cream ⁶Statistically significant when compared to Active cream | |

The above results demonstrate that a topical composition comprising a lipophilic carrier composition of the present invention that was produced using a presolubilized *Cnidium monnieri* fruit extract provided better protection against chemically-induced skin erythema than the same composition lacking certain ingredients present in the inventive composition.

### EXAMPLE 6B:

### SKIN SOOTHING AND REPAIRING EFFECT AFTER EXPOSURE TO CHEMICAL AGENT

In another experiment, some skin areas were treated only upon removal of the SLS-containing Finn chambers (Day 12). In this case, skin was post-treated with one application (2 mg/cm²) of the Placebo cream prepared according to Formula 1 or the *Echium Plantaginium* Active Cream with *Cnidium Monnieri* Fruit Extract prepared according to Formula 4. The repairing effect of the treatments was assessed for skin barrier function and for skin erythema at time 30 minutes, 1 hour, 2 hours and 24 hours after the topical application of the cream formulations. The skin barrier function and erythema were assessed as described in Example 6A above. Results are shown in Tables 9 and 10 and reveal that applying the active cream formulation after the chemical stress with SLS significantly reduces the extent of skin barrier damage. This demonstrates the therapeutic action of the active cream formulation in promoting skin barrier function. The data on skin erythema in Table 10 also demonstrate that the therapeutic application of the active cream formulation can help repair chemically-induced skin erythema.

**Table 9. Effect of a topical composition incorporating a presolubilized Cnidium monnieri fruit extract in a lipophilic carrier composition comprising Echium plantagineum seed oil on chemically-induced skin barrier damage when cream formulations are applied after chemical damage. The therapeutic effect was measured at various time periods after the cream formulation application.**

| | Reduction of chemically-induced skin increase in TEWL upon product application (%) | | | |
|---|---|---|---|---|
| | After 30 min | After 1 hour | After 2 hour | After 24 hour |
| Control (untreated skin) | -1.3 | -6.4 | -11.2 | -23.2 |
| Base cream: Formula 1 | -8.1 | -8.4 | -13.2 | -20.6 |
| Active cream: Formula 4 | -18.5 (p=0.005)¹ | -24.4 (p=0.007)¹ | -24.5 (p=0.006)¹ | -28.8 (p=0.009)¹ |

| | | | | |
|---|---|---|---|---|
| ¹Statistically significant when compared to Placebo cream | | | | |

**Table 10. Effect of a topical composition incorporating a presolubilized Cnidium monnieri fruit extract in a lipophilic carrier composition comprising Echium plantagineum seed oil on chemically-induced skin erythema when cream formulations are applied after chemical damage. The therapeutic effect was measured at various time periods after the cream formulation application**

| | Reduction of chemically-induced skin erythema upon product application (%) | | | |
|---|---|---|---|---|
| | After 30 min | After 1 hour | After 2 hour | After 24 hour |
| Control (untreated skin) | 3.7 | 4.1 | 4.4 | 2.4 |
| Base cream: Formula 1 | -4.3 | -5.5 | -5.6 | -6.1 |
| Active cream: Formula 4 | -5.2 (p=0.463)² | -6.7 (p=0.510)² | -7.0 (p=0.394)² | -9.2 (p=0.032)¹ |

| | | | | |
|---|---|---|---|---|
| ¹Statistically significant when compared to Placebo cream ²Not statistically significant when compared to Placebo cream | | | | |

The data shown in Tables 9 and 10 demonstrate that a topical composition comprising a lipophilic carrier composition of the present invention that was produced using a presolubilized *Cnidium monnieri* fruit extract provided better protection against an increase in TEWL as well as better protection against chemically-induced skin erythema than the same composition lacking certain ingredients present in the inventive composition.

### EXAMPLE 7:

### RIBES NIGRUM-BASED ACTIVE CREAM WITH PAEONIA SUFFRUTICOSA ROOT EXTRACT

Formulas 1-4 as described above were prepared wherein the lipophilic bioactive botanical extract was *Paeonia suffruticosa* root extract and the vegetable oil was *Ribes nigrum* seed oil.

### EXAMPLE 7A: SKIN PROTECTIVE, SOOTHING EFFECT UPON UV EXPOSURE

The skin protective, soothing and repairing effect of a cosmetic topical product incorporating the presolubilized *Paeonia siffruticosa* root extract in a lipophilic carrier composition comprising *Ribes nigrum* seed oil upon UV exposure (UVA+B using a solar simulator) was evaluated by applying topical treatments (2 mg/cm²) of each of Formulas 1 through 4.

The UV exposure intensity was calibrated to induce 1.5 MED. 1 MED, or Minimal Erythema Dose, corresponds to the minimum amount of UVB radiation required to produce redness 24 hours after skin exposure. The test was carried out on a panel of 10 healthy volunteers. Selected skin areas were kept untreated and unexposed to UV as controls. Other skin areas were treated with the relevant formulation for a period of 10 days (from Day 1 to Day 10) preceding UV exposure.

On Day 11, specific skin sites were exposed to UV. On Day 12, 20±4 hours after UV exposure, skin erythema and the skin barrier function were assessed to measure the protective effect of the treatments. The skin barrier function was assessed by the measurement of trans-epidermal water loss (TEWL) using the TEWAMETER 300^{®} apparatus (Courage+Khazaka, electronic GmbH) and skin erythema was measured using the MEXAMETER^{®} MX 18 apparatus (Courage+Khazaka, electronic GmbH). The results are reported in Tables 11 and 12.

The application of the cosmetic topical product incorporating the presolubilized *Paeonia suffruticosa* root extract in a *Ribes nigrum* seed oil prior to the application of UV light resulted in a reduction of the UV-induced increase in TEWL (Table 11). The most favorable results were observed for the active cream formulation, which was produced by using the solubilization system of the present invention to presolubilized the *Paeonia suffruticosa* root extract, followed by addition of the *Ribes nigrum* seed oil to form a lipophilic carrier composition comprising both the *Paeonia suffruticosa* root extract and the *Ribes nigrum* seed oil. This lipophilic carrier composition was incorporated into the base formulation to give the active cream. These results demonstrate that the active cream formulation can protect against UV-induced skin barrier function loss.

The measurement of skin erythema (Table 12) demonstrated that the application of the active cream formulation can protect from UV-induced skin erythema.

**Table 11. Effect of a topical composition incorporating a presolubilized Paeonia suffruticosa root extract in a lipophilic carrier composition comprising Ribes nigrum (black currant) seed oil on the prevention of UV-induced skin barrier damage - reduction of TEWL increase when cream formulations are applied as a preventive treatment.**

| | UV-induced increase in TEWL (%)¹ |
|---|---|
| Control (untreated skin) | 79.5 |
| Formula 1: placebo base cream | 81.2 |
| Formula 2: Formula 1 with *Ribes nigrum* (black currant) seed carrier oil | 51.9 (p=0.001) vs Placebo⁵ |
| | (p=0.612) vs Active cream⁶ |
| Formula 3: Formula 2 with antioxidants and solubilization system | 50.7 (p=0.002) vs Placebo³ |
| | (p=0.705) vs Active cream⁴ |
| Active Cream Fonnula 4: Formula 3 with presolubilized *Paeonia suffruticosa* root extract | 48.0 (p<0.002)² |

| | |
|---|---|
| ¹Compared to non-irradiated skin (baseline) ²Statistically significant when compared to Placebo cream ³Statistically significant when compared to Placebo cream ⁴Non-statistically significant when compared to Active cream ⁵Statistically significant when compared to Placebo cream ⁶Non-statistically significant when compared to Active cream | |

**Table 12. Effect of a topical composition incorporating a presolubilized Paeonia suffruticosa root extract in a lipophilic carrier composition comprising Ribes nigrum (black currant) seed oil on UV-induced skin erythema when applied as a cream formulation as a preventive treatment.**

| | UV-induced increase in skin erythema (%)¹ |
|---|---|
| Control (untreated skin) | 62.1 |
| Formula 1: base cream | 57.3 |
| Formula 2: Formula 1 with *Ribes nigrum* (Black Currant) seed carrier oil | 44.4 (p=0.012) vs Placebo⁵ |
| | (p=0.127) vs Active cream |
| Formula 3: Formula 2 with antioxidants and solubilization system | 36.5 (p=0.014) vs Placebo³ |
| | (p=0.470) vs Active cream⁴ |
| Active Cream Formula 4: Formula 3 with presolubilized *Paeonia suffruticosa* root extract | 33.0 (p<0.002)² |

| | |
|---|---|
| Compared to non-irradiated skin (baseline) ²Statistically significant when compared to Placebo cream ³Statistically significant when compared to Placebo cream ⁴Non-statistically significant when compared to Active cream ⁵Statistically significant when compared to Placebo cream ⁶Non-statistically significant when compared to Active cream | |

### EXAMPLE 7B: SKIN SOOTHING AND REPAIRING EFFECT AFTER UV EXPOSURE

In another experiment, some skin areas were treated only after exposure of the skin to UV (Day 12). In this case, skin was post-treated with one application (2 mg/cm²) of the Placebo cream prepared according to Formula 1 or the Active Cream prepared according to Formula 4. The repairing effect of the treatments was assessed for skin barrier function and for skin erythema at time 30 minutes, 1 hour, 2 hours and 24 hours upon the topical application of the cream formulations. The skin barrier function and erythema were assessed as described above. Results are reported in Tables 13 and 14 and show that applying the active cream formulation after UV exposure significantly reduces the extent of skin barrier damage (Table 13). This provides evidence for the therapeutic action of the active cream formulation in promoting skin barrier function. The measurement of skin erythema (Table 14) also demonstrated that the therapeutic application of the active cream formulation can help repair damage from UV-induced skin erythema.

**Table 13. Effect of a topical composition incorporating a presolubilized Paeonia suffruticosa root extract in a lipophilic carrier composition comprising Ribes nigrum (black currant) seed oil on UV-induced skin barrier damage when cream formulations were applied after UV - therapeutic effect measured at various time periods after the cream formulation application.**

| | Reduction of UV-induced skin increase in TEWL upon product application (%) | | | |
|---|---|---|---|---|
| | After 30 min | After 1 hour | After 2 hour | After 24 hour |
| Control (untreated skin) | -0.6 | 1.2 | -0.8 | -6.2 |
| Placebo Base cream: Formula 1 | -10.1 | -9.8 | -12.3 | -10.2 |
| Active cream: Formula 4 | -16.7 (p=0.003)¹ | -18.2 (p=0.001)¹ | -19.7 (p=0.003)¹ | -28.7 (p<0.001)¹ |

| | | | | |
|---|---|---|---|---|
| ¹Statistically significant when compared to Placebo cream | | | | |

**Table 14. Effect of a topical composition incorporating a presolubilized Paeonia suffruticosa root extract in a lipophilic carrier composition comprising Ribes nigrum seed oil on UV-induced skin erythema wen cream formulations are applied after UV - therapeutic effect measured at various time periods after the cream formulation application.**

| | Reduction of UV-induced skin erythema upon product application (%) | | | |
|---|---|---|---|---|
| | After 30 min | After 1 hour | After 2 hour | After 24 hour |
| Control | 0.7 | -1.1 | -3.3 | -11.0 |
| (untreated skin) | | | | |
| Placebo base cream: Formula 1 | -6.2 | -9.1 | -10.3 | -11.7 |
| Active cream: Formula 4 | -8.7 (p=0.046)¹ | -12.1 (p=0.010)¹ | -16_{.}3 (p=0.008)¹ | -19.5 (p=0.007)¹ |

| | | | | |
|---|---|---|---|---|
| statistically significant when compared to Placebo cream | | | | |

### EXAMPLE 7C: REDUCTION OF THE APPEARANCE OF AGE SPOTS (MELASMA)

Another experiment demonstrated the efficacy of a cosmetic topical product incorporating the presolubilized *Paeonia suffruticosa* root extract in a lipophilic carrier composition comprising *Ribes nigrum* seed oil in reducing the appearance of age spots (also known as brown spots, liver spots, melasma, solar lentigo, freckles, senile freckles, lengitines or chloasma "mask of pregnancy"). Age spots are hyperpigmented skin areas mat may arise from over UV-expasure, in pregnant women, or in subjects undergoing hormonal replacement therapies. Their visual appearance is due to an accumulation of lnelallocytes and/or an excessive production of melanin pigments and this phenomenon becomes more apparent with increasing age. The efficacy of the cosmetic topical products disclosed herein for the treatment of age spots was evaluated by applying topical treatments (2 mg/cm²) of each of Formula 1 (Placebo cream) and Formula 4 (Active cream).

The test was carried out on a panel of 15 healthy volunteers with visible age spots. Formulas 1 and 4 were tested as a split-face clinical protocol where each Formula was applied on separate sides of the face down up to the upper chest area, twice daily. Evaluation of age spot appearance was measured at Day 0 (baseline) and after 30 (Day 30) and 60 days (Day 60) of product applications. The MEXAMETER^{®} MIX 18 apparatus (Courage+Khazaka, electronic GmbH) was used to measure the age spot color based on specific light wave-length absorption by melanin-related chromophores. Results are expressed as variation of the melanin index. The Spectrophotometer CM-700d (Konica Minolta Optics, Inc) was chosen to measure the age spot color intensity by computing specific light wave-length reflection of *L* * parallleter (skin brightness) and *b** parameter (variation from blue to yellow color) as the Individual Typology Angle (ITA°). An increase in ITA° is indicative of a color intensity reduction. A separate individual measurement of the *L** parameter was also performed to assess variations in general skin color lightness.

As a more visual assessment of the effect the treatments, a skilled dermatologist evaluated, in a blind fashion, the visual reduction of age spot appearance and the increase in skin complexion (color homogeneity). The evaluation criteria were as follows:

**Table 15**

| Dermatologist clinical evaluation at Day 30 and Day 60 in comparison to baseline | Score |
|---|---|
| No variation | 1 |
| Slight improvement | 2 |
| Moderate improvement | 3 |
| Remarkable improvement | 4 |

Topical applications of the Active cream statistically reduced the melanin index (age spot pigment density) and increased dire lightening of age spots present in the face, neck, and upper chest areas (Table 16 and 17, respectively). Furthermore, treatment with the Active cream statistically improved the general skin lightness (Table 18). Dermatologist assessments have shown that a reduction of age spot color appearance (Table 19) and an improvement in skin complexion (Table 20) can be visually observed already after 30 days of treatment. Those results demonstrate that topical applications of a cosmetic product incorporating the presolubilized *Paeonia suffruticosa* root extract in a lipophilic carrier composition comprising *Ribes nigrum* seed oil reduces the color pigmentation and the visual appearance of age spots. Furthermore, a general improvement in skin complexion could be observed.

**Table 16. Effect of a topical composition incorporating a presolubilized Paeonia suffruticosa root extract in a lipophilic carrier composition comprising Ribes nigrum seed oil on age spot melanin index - MEXAMETER^{®} MX 18.**

| | Variation in melanin index upon product application in comparison to baseline (%) | | | |
|---|---|---|---|---|
| | Day 30 | | Day 60 | |
| Treatment | Placebo cream | Active cream | Placebo cream | Active cream |
| Melanic index variation (%) | -2.5 | -14.4 | -1.5 | -18.1 |
| p value | N/A | p=0.025¹ | N/A | p=0.004¹ |

| | | | | |
|---|---|---|---|---|
| statistically significant when compared to Placebo cream | | | | |

**Table 17. Effect of a topical composition incorporating a presolubilized Paeonica suffruticosa root extract in a lipophilic carrier composition comprising Ribes nigrum seed oil on age spot color intensity reduction (ITA°) - Spectrophotometer CM-700d.**

| | ITA° variation upon product application in comparison to baseline (%) | | | |
|---|---|---|---|---|
| | Day 30 | | Day 60 | |
| Treatment | Placebo cream | Active cream | Placebo cream | Active cream |
| ITA° variation (%) | -2.6 | +12_{.}4 | -3.7 | +20.1 |
| p value | N/A | p=0.019¹ | N/A | p=0.002¹ |

| | | | | |
|---|---|---|---|---|
| ¹Statistically significant when compared to Placebo cream | | | | |

**Table 18. Effect of a topical composition incorporating a presolubilized Paeonia suffruticosa root extract in a lipophilic carrier composition comprising Ribes nigrum seed oil on face, neck and upper chest skin area color brightness (L*) - Spectrophotometer CM-700d.**

| | *L** Variation upon product application in comparison to baseline (%) | | | |
|---|---|---|---|---|
| | Day 30 | | Day 60 | |
| Treatment | Placebo cream | Active cream | Placebo cream | Active cream |
| *L** parameter variation (%) | -0.7 | +2.5 | -0.5 | +4.2 |
| p value | N/A | p=0.003¹ | N/A | p<0.001¹ |

| | | | | |
|---|---|---|---|---|
| ¹Statistically significant when compared to Placebo cream | | | | |

**Table 19. Effect of a topical composition incorporating a presolubilized Paeonia suffruticosa root extract in a lipophilic carrier composition comprising Ribes nigrum seed oil on age spot visual appearance - dermatologist evaluation.**

| | Proportion of subjects for which a reduction in the visual appearance of age spots was observed (%) | | | |
|---|---|---|---|---|
| | Day 30 | | Day 60 | |
| Treatment | Placebo cream | Active cream | Placebo cream | Active cream |
| Proportion of subjects (%) | 0.0 | 26.7 | 6.7 | 53.3 |

**Table 20. Effect of a topical composition incorporating a presolubilized Paeonia suffruticosa root extract in a lipophilic carrier composition comprising Ribes nigrum seed oil on the visual assessment of skin complexion - dermatologist evaluation.**

| | Proportion of subjects for which an improvement of skin complexion was visually observed (%) | | | |
|---|---|---|---|---|
| | Day 30 | | Day 60 | |
| Treatment | Placebo cream | Active cream | Placebo cream | Active cream |
| Proportion of subjects (%) | 0.0 | 13.3 | 6.7 | 33.3 |

## Claims

1. A lipophilic carrier composition comprising a lipophilic bioactive botanical extract, a vegetable oil, and a solubilization system, where the vegetable oil is resistant to oxidation.

2. The composition of claim 1 where the Rancimat induction time for the composition according to ISO Method no. 6886-2006 is greater than 3 hours.

3. The composition of claim 1 comprising an antioxidant.

4. The composition of claim 1 where the solubilization system comprises a branched, long chain alcohol and one or more fatty acid esters of a branched, long chain alcohol.

5. The composition of claim 4 where the branched, long chain alcohol is octyldodecanol.

6. The composition of claim 5 where the one or more fatty acid esters is a mixture of octyldodecyl oleate and octyldodecyl stearoyl stearate.

7. The composition of claim 6 where the solubilization system further comprises ethanol.

8. The composition of claim 1 where the vegetable oil is *Echium plantagineum* seed oil or *Ribes nigrum* seed oil.

9. The composition of claim 1 where the lipophilic bioactive botanical extract is *Cnidium monnieri* fruit extract or *Paeonia suffruticosa* root extract.

10. The composition of claim 1 comprising *Paeonia suffruticosa* root extract, *Ribes nigrum* seed oil, ethanol, octyldodecanol, octyldodecyl oleate, and octyldodecyl stearoyl stearate.

11. A lipophilic antioxidant composition comprising an antioxidant, a vegetable oil, and a solubilization system, and not comprising a lipophilic bioactive botanical extract, where the vegetable oil is resistant to oxidation.

12. The composition of claim 11 where the Rancimat induction time for the composition according to ISO Method no. 6886-2006 is greater than 3 hours.

13. The composition of claim 11 where the solubilization system comprises a branched, long chain alcohol and one or more fatty acid esters of a branched, long chain alcohol.

14. The composition of claim 13 where the branched, long chain alcohol is octyldodecanol.

15. The composition of claim 14 where the one or more fatty acid esters is a mixture of octyldodecyl oleate and octyldodecyl stearoyl stearate.

16. The composition of claim 15 where the solubilization system further comprises ethanol.

17. The composition of claim 11 where the vegetable oil is *Echium plantagineum* seed oil or *Ribes nigrum* seed oil.

18. A topical cosmetic formulation comprising the lipophilic carrier composition according to any of the preceding claims.

19. A method of treating or protecting skin against erythema or skin barrier function loss comprising applying the topical cosmetic formulation of claim 18 to skin that has suffered or is at risk of suffering radiation or chemical stress.

20. A method of treating age spots comprising applying the topical cosmetic formulation of claim 18 to skin containing age spots.
